# EUROPEAN PATENT APPLICATION

(11) **EP 4 759 275 A1**
(43) Date of publication of application: **17.06.2026**
(21) Application number: 24897966.8
(22) Date of filing: 15.11.2024
(51) Int. Cl.: A61F 2/90, A61F 2/82

(54) **STENT**

(30) Priority: 27.11.2023 KR 20230166669
(71) Applicant: BCM Co., Ltd., Goyang-si, Gyeonggi-do 10208 (KR)
(72) Inventor: MYUNG, Byung Cheol, Paju-si, Gyeonggi-do 10894 (KR)
(74) Representative: Porta & Consulenti Associati S.p.A.
(86) International application number: PCT/KR2024/018028
(87) International publication number: WO 2025/116370

(57) **Abstract**

The present invention relates to a stent having a pull wire, wherein, when an outer tube is pulled at a lesion site, the pull wire in point contact therewith reduces resistance, allowing the outer tube to be pulled smoothly, and the pull wire prevents the thickness of a portion of the outer tube from being increased by the length of the pull wire, and, more specifically, to a stent comprising: a cylindrical body in which shape memory alloy wires are woven or crossed in a mesh form to have a hollow cylindrical shape, thereby forming a plurality of space portions between the wires, and which has a plurality of bent portions formed along the circumference thereof at both ends; and a pull wire provided at one end of the cylindrical body, wherein the pull wire consists of a single strand, wherein one end of the pull wire is tied to the wire of one bent portion to form a first knot portion, and the opposite end of the pull wire is woven through the space portions of the plurality of bent portions along the circumference of the cylindrical body and positioned adjacent to the first knot portion, and then is allowed to protrude outward from the cylindrical body, and the opposite end of the pull wire protruding outward from the cylindrical body is tied, thereby forming a second knot portion.

## Description

### Technical Field

The present disclosure relates to a stent that is deployed at a lesion site where stenosis or occlusion has occurred in a lumen within a body, and that can be easily removed from the lumen within the body through a pull string after a predetermined period of time has elapsed.

### Background Art

Generally, when a lesion site causing stenosis or occlusion occurs in a lumen within a body, such as the esophagus, duodenum, biliary tract of a human body, a problem arises in that the inherent function of the lumen within the body for transporting body fluids is degraded.

For this reason, a stent is inserted into the position of the lesion site causing stenosis or occlusion to expand the narrowed lumen within the body.

In addition, after completion of a procedure at the lesion site, when a predetermined period of time has elapsed, the stent is required to be removed from the lumen within the body.

In this regard, Patent Document 1 provides a hook structure of a pull string for removing a stent, the stent (10) including a cylindrical body (14) having space portions (12) formed by intertwining or zigzag-crossing one or more strands of shape memory alloy wires (11) with each other and having multiple bent ends (13) formed along a circumference thereof at opposite ends thereof, wherein a circumferential pull string (21) is formed by intertwining the pull string in a zigzag manner to form a circular band shape at the bent ends (13) of the cylindrical body (14) of the stent (10), and opposite ends of the circumferential pull string (21) extend longitudinally and are tied together to form a hook (22), thereby forming the pull string (20).

However, in the case of Patent Document 1, as illustrated in FIG. 1, when the stent (10) is compressed in an outer tube (2a) of a stent delivery system (2) and mounted in a mounting space (2d') formed in an inner tube (2d), a pair of pull strings (20) protruding outward from the stent (10) are in long surface contact with an inner surface of the outer tube (2a).

Due to this, when the outer tube (2a) is pulled during a procedure of placing the stent (10) at a lesion site, resistance is generated by the pull strings (20) that are in surface contact, and thus the outer tube (2a) may not be smoothly pulled. That is, the placement procedure of the stent (10) may not be properly performed.

In addition, when the outer tube (2a) is pulled with a greater external force because the outer tube (2a) is not smoothly pulled due to the pull strings (20) that are in surface contact, the outer tube (2a) may be torn or ruptured.

Further, since the pair of pull strings (20) is positioned between the outer tube (2a) and the stent (10), a thickness of a portion of the outer tube (2a) is increased by a length (L) of the pair of pull strings (20).

As a result, the outer tube (2a) having the increased thickness may generate resistance within the lumen in the body, making movement difficult, or may not easily pass through a narrow lesion site.

### [Prior Art Document]

### [Patent Document]

Patent Document 1: Korean Patent Application Publication No. 10-2010-0119068.

### Disclosure

### Technical Problem

Accordingly, an objective of the present disclosure is to provide a stent having a pull string different from that of the related art, wherein when an outer tube is pulled at a lesion site, resistance thereof is reduced by the pull string in point contact therewith, thereby allowing the outer tube to be easily pulled and facilitating deployment of the stent, and wherein a thickness of a portion of the outer tube is prevented from being increased by a length of the pull string, thereby facilitating movement of the outer tube within a lumen in a body and passage of the outer tube through a narrow lesion site.

### Technical Solution

In order to accomplish the above objectives, the present disclosure provides a stent including a cylindrical body formed such that shape memory alloy wires are woven or crossed in a mesh form to have a hollow cylindrical shape so that multiple space portions are formed between the wires and multiple bent portions are formed along a circumference of the cylindrical body at each of opposite ends thereof, and a pull string installed at one end of the cylindrical body, the stent being configured to be deployed at a lesion site where stenosis or occlusion has occurred in a lumen in a body and to expand the lesion site, wherein the pull string consists of a single strand, one end of the pull string is tied to the wire of one of the bent portions to form a first knotted portion, an opposite end of the pull string is woven through the spaces of the multiple bent portions along the circumference of the cylindrical body so as to be positioned adjacent to the first knotted portion, and then protrudes outward from the cylindrical body, and the opposite end of the pull string protruding outward from the cylindrical body is tied to form a second knotted portion.

### Advantageous Effects

According to the present disclosure, when an outer tube is pulled at a lesion site, resistance is reduced by a second knot portion of a pull string that is in point contact with an inner surface of the outer tube, so that the outer tube is smoothly pulled, thereby allowing a stent placement procedure to be performed more smoothly than in the related art.

In addition, since it is not necessary to pull the outer tube with a greater external force, the outer tube is prevented from being torn or cut due to the greater external force.

In addition, it is not necessary to use an outer tube having a large diameter in order to reduce resistance caused by the pull string, thereby preventing difficulty in movement of the outer tube within a lumen in a body and difficulty in passage of the outer tube through a narrow lesion site due to the increase in diameter of the outer tube.

According to the present disclosure, a single pull string rather than a pair of pull strings is positioned between the outer tube and the stent, thereby making thickness of a portion of the outer tube, at which the pull string is positioned, smaller compared with the related art.

That is, a portion of the outer tube does not become thicker by a length of the pull string than in the prior art.

For this reason, during deployment of the stent, the outer tube that does not become thicker than in the prior art has reduced resistance within the lumen in the body, so that movement of the outer tube is more easily performed than in the prior art, and passage of the outer tube through a narrow lesion site is more easily performed than in the prior art.

According to the present disclosure, when the stent is removed after a predetermined period of time has elapsed after completion of deployment procedure of the stent at the lesion site, the stent can be removed by pulling the second knotted portion by using a gripping member such as forceps.

For this reason, slipping of the gripping member such as forceps on the pull string formed of a slippery material is prevented.

According to the present disclosure, since the multiple second knotted portions are formed on the pull string, a greater number of the second knotted portions can be grasped by a gripping member such as forceps.

For this reason, the multiple second knotted portions are caught by the gripping member such as forceps, thereby further preventing the gripping member from slipping on the pull string formed of a slippery material, and allowing a pulling external force to be increased so that the stent can be rapidly removed.

In addition, since one of the multiple second knotted portions can be grasped by the gripping member such as forceps at any position of the pull string, a second knotted portion (220) positioned at a location suitable for removal of the stent can be selected from among the multiple second knotted portions (220) and pulled by the gripping member such as forceps, thereby enabling the stent to be removed.

According to the present disclosure, since a gap is formed between one end of the cylindrical body of the stent and the second knotted portion adjacent thereto, when the compressed stent is expanded, an inlet of the stent is prevented from being caught by the second knotted portion and failing to expand to an original state thereof.

For this reason, expansion of the lesion site by the stent according to the present disclosure is smoothly performed.

### Description of Drawings

FIGS. 1 and 2 are a view showing a state of use of a conventional stent and a partially enlarged view thereof,
FIGS. 3 and 4 are a front view and a plan view of a stent according to an embodiment of the present disclosure,
FIGS. 5 to 7 are a partially enlarged view of FIG. 3 and views showing a process in which a first knotted portion is formed,
FIGS. 8 to 10 are a partially enlarged view of FIG. 3 and views showing a process in which a second knotted portion is formed,
FIG. 11 is a view showing a state of use of the stent illustrating a reason why a gap is formed between a cylindrical body and the second knotted portion of the stent according to an embodiment of the present disclosure,
FIG. 12 is a partially enlarged sectional view of FIG. 4, and
FIGS. 13 to 21 are views showing a state of use of the stent according to an embodiment of the present disclosure.

### Best Mode

Accordingly, an embodiment of the present disclosure as described above will be described in detail with reference to the accompanying drawings as follows.

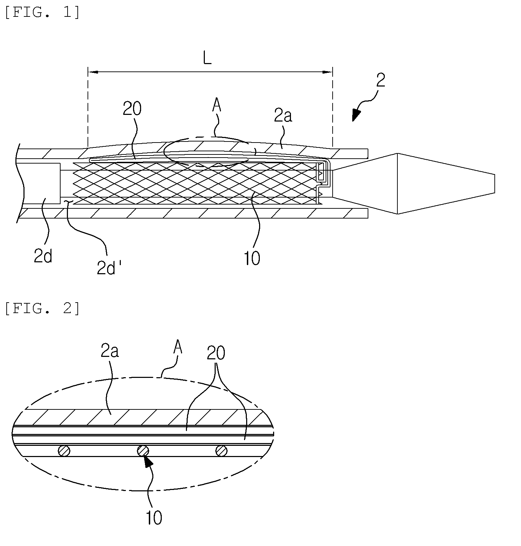

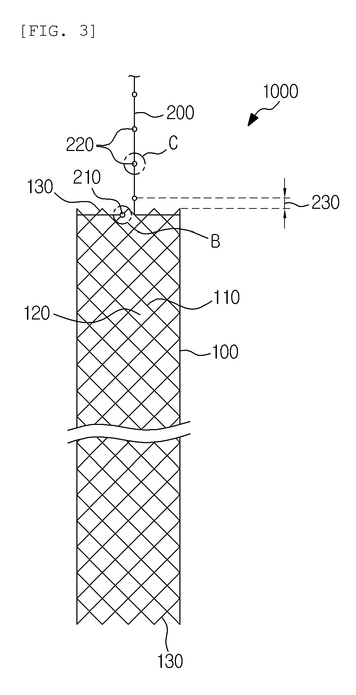

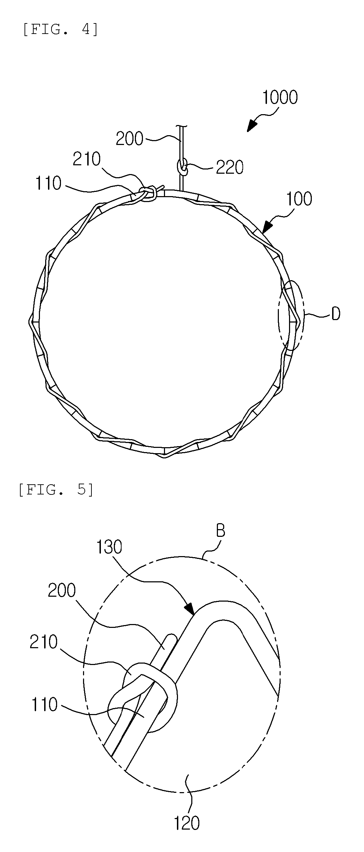

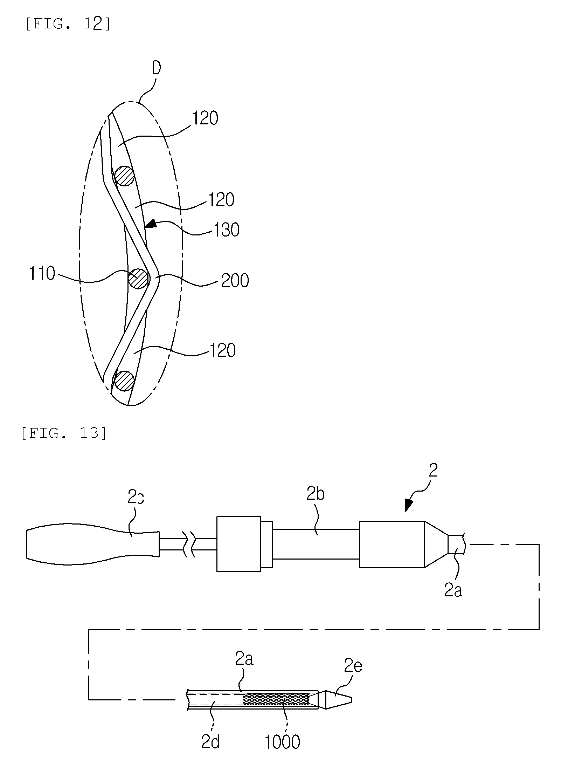

As illustrated in FIGS. 1 to 21, a stent (1000) according to an embodiment of the present disclosure is inserted into a lumen (1) in a body such as an esophagus, a duodenum, or a bile duct of a human body by means of a stent delivery system (2), and is used when expanding a lesion site (1a) in which stenosis or occlusion has occurred in the lumen (1).

Here, the stent delivery system (2) includes a first handle (2b) to which an outer tube (2a) moving along the lumen (1) in the body is connected; a second handle (2c) configured to move from the first handle (2b); an inner tube (2d) connected to the second handle (2c) and inserted into the outer tube (2a) so as to be movable inside the outer tube (2a), the inner tube (2d) having, at one side thereof, a mounting space (2d') in which the stent (1000) of the present disclosure is mounted in a compressed state in the outer tube (2a); and a guide tip (2e) connected to the inner tube (2d) and exposed from the outer tube (2a).

The stent (1000) includes a cylindrical body (100) in which wires (110) made of a shape memory alloy are woven or crossed in a mesh form to have a hollow cylindrical shape such that multiple space portions (120) are formed between the wires (110) and multiple bent portions (130) are formed along a circumference thereof at each of opposite ends thereof, and a pull string (200) installed at one end of the cylindrical body (100).

The pull string (200) consists of a single strand and is made of, for example, a nylon material, and one end of the pull string (200) is tied to the wire (110) of one of the bent portions (130) to form a first knotted portion (210).

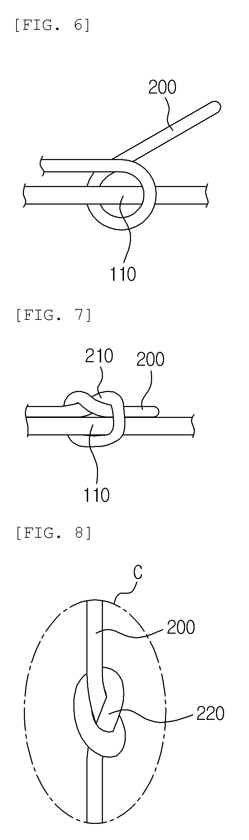

Describing in detail a process of forming the first knotted portion (210), as shown in FIGS. 6 and 7, one end of the pull string (200) passes through the space portion (120) of one bent portion (130) and is twisted so that at least one loop penetrating the wire (110) is formed, and when the loop is passed through the one end of the pull string (200), the loop is reduced in size, whereby the one end of the pull string (200) is tied to the wire (110) to form the first knotted portion (210).

The pull string (200) has an opposite end thereof woven through the space portions (120) of the multiple bent portions (130) along the circumference of the cylindrical body (100) so as to be positioned adjacent to the first knotted portion (210), and then protruding outward from the cylindrical body (100), and the opposite end of the pull string (200) protruding outward from the cylindrical body (100) is tied to form a second knotted portion (220).

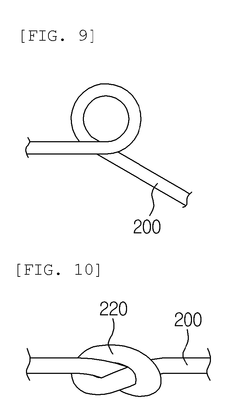

Describing in detail a process of forming the second knotted portion (220), as shown in FIGS. 9 and 10, the opposite end of the pull string (200) protruding outward from the cylindrical body (100) is twisted to form at least one loop, and when the loop is passed through the opposite end of the pull string (200), the loop is reduced in size, whereby the opposite end of the pull string (200) is tied to form the second knotted portion (220).

Here, the second knotted portion (220) includes multiple second knotted portions formed at predetermined intervals along a longitudinal direction of the pull string (200) protruding outward from the cylindrical body (100).

In addition, a gap (230) is formed between the one end of the cylindrical body (100) and the second knotted portion (220) adjacent thereto.

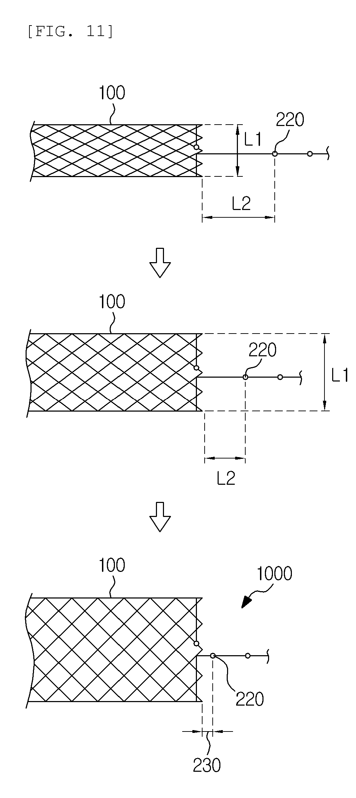

As illustrated in FIG. 11, describing in detail a reason why the gap (230) is formed, when the stent (1000) of the present disclosure is compressed in the outer tube (2a) of the stent delivery system (2) and mounted in the mounting space (2d') of the inner tube (2d), a diameter length (L1) of the cylindrical body (100) is shorter than a length (L2) between one end of the cylindrical body (100) and the second knotted portion (220) adjacent thereto, and when the stent (1000) of the present disclosure is deployed at the lesion site (1a), the diameter length (L1) of the cylindrical body (100) is longer than the length (L2) between the one end of the cylindrical body (100) and the second knotted portion (220) adjacent thereto. In this case, in order to prevent an inlet of the expanding cylindrical body (100), that is, an inlet of the stent (1000), from being caught by the second knotted portion (220), the gap (230) is formed between the one end of the cylindrical body (100) and the second knotted portion (220) adjacent thereto.

Here, the gap (230) is formed to be 3 mm to 10 mm.

The operation of the stent (1000) according to an embodiment of the present disclosure will now be described.

As illustrated in FIGS. 1 to 21, the stent (1000) according to the embodiment of the present disclosure is compressed within the outer tube (2a) of the stent delivery system (2) and mounted in the mounting space (2d') of the inner tube (2d).

Then, the second knotted portion (220) of the pull string (200), which protrudes outward from the stent (1000), is in point contact with an inner surface of the outer tube (2a). Since the pull string (200) consists of a single strand and is positioned between the outer tube (2a) and the cylindrical body (100) of the stent (1000), a thickness of a portion of the outer tube (2a) in which the pull string (200) is located is not increased.

Of course, even when the pull string (200) is positioned inside the cylindrical body (100) of the stent (1000), rather than outside the cylindrical body (100), the thickness of the portion of the outer tube (2a) where the pull string (200) is located is not increased, because the pull string (200) consists of a single strand.

In addition, after checking a location of the lesion site (1a) by endoscopy, the first and second handles (2b, 2c) of the stent delivery system (2) are advanced so that the inner tube (2d) and the outer tube (2a) are advanced along the lumen (1) within the body.

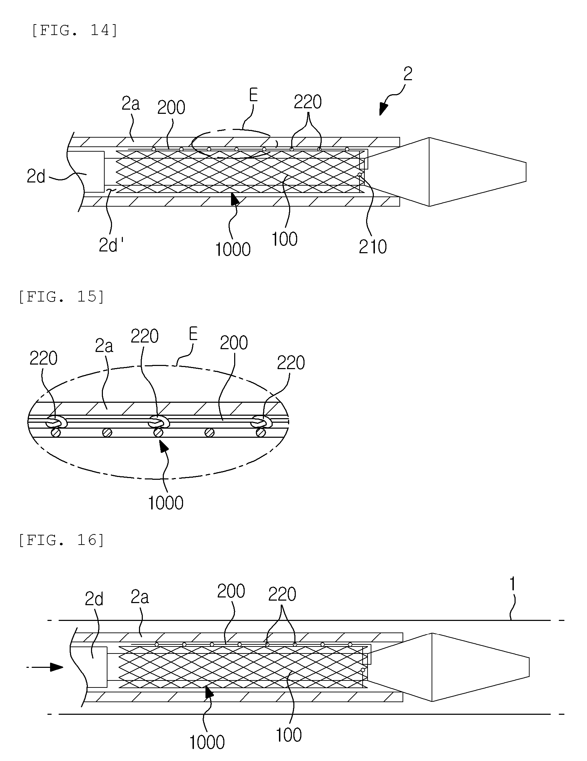

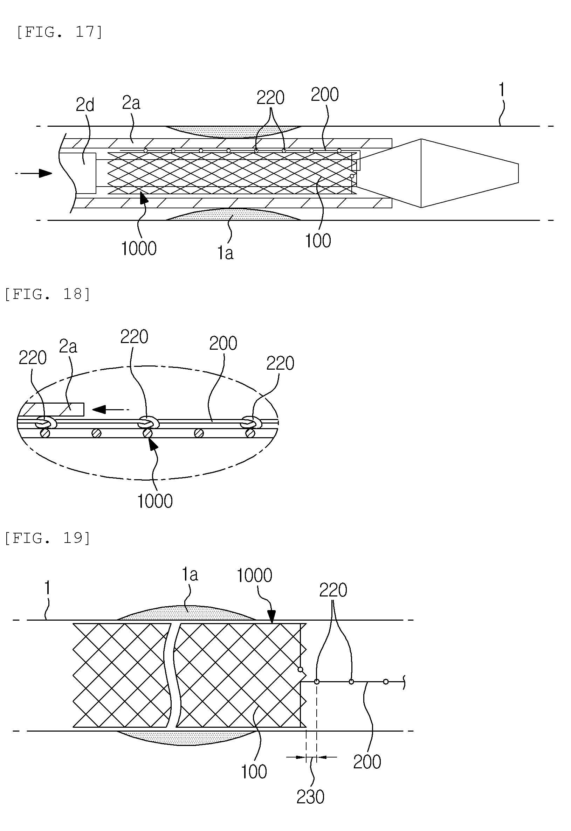

Here, since a thickness of the outer tube (2a) is not increased by the pull string (200) consisting of a single strand, as illustrated in FIG. 16, the outer tube (2a) is easily moved to the lesion site (1a) because catching of the outer tube (2a) occurring in the lumen (1) within the body during movement thereof, that is, resistance thereof is reduced, and as illustrated in FIG. 17, the outer tube (2a) easily passes through the narrow lesion site (1a) because catching of the outer tube (2a) occurring when passing through the narrow lesion site (1a), that is, resistance thereof is reduced.

As illustrated in FIG. 18, since the second knotted portion (220) is in point contact with the inner surface of the outer tube (2a), resistance generated when pulling the outer tube (2a) through the first handle (2b) at the lesion site (1a) is reduced, thereby allowing the outer tube (2a) to be easily pulled.

For this reason, the stent (1000) of the present disclosure, which has been compressed within the outer tube (2a), expands without delay.

As illustrated in FIG. 19, since the gap (230) is formed between the one end of the cylindrical body (100) and the second knotted portion (220) adjacent thereto, the inlet of the expanding cylindrical body (100), that is, the inlet of the stent (1000), is not caught on the second knotted portion (220).

For this reason, the stent (1000) of the present disclosure is easily expanded to expand the lesion site (1a) .

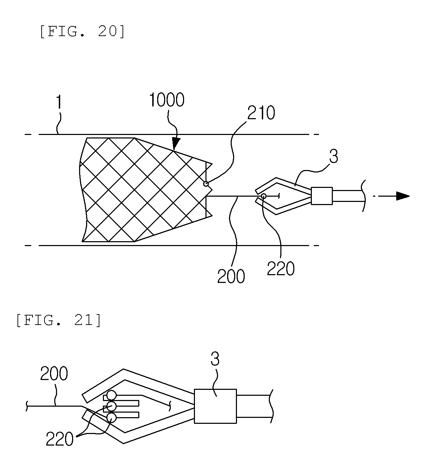

As illustrated in FIG. 20, when a predetermined period of time has elapsed after completion of deployment of the stent (1000) at the lesion site (1a) where stenosis or occlusion has occurred in the lumen (1) within the body, the removal of the stent (1000) is required. In this case, one of the multiple second knotted portions (220) of the pull string (200) is grasped and pulled by using a gripping member (3) such as forceps.

Then, while the first knotted portion (210) fixed to the bent portion (130) of the stent (1000) is pulled by the pulled second knotted portion (220), the inlet of the stent

(1000) is contracted, thereby allowing the stent (1000) to be easily withdrawn from the lumen (1) within the body.

As illustrated in FIG. 21, at least two or more second knotted portions (220) may be grasped and pulled by the gripping member (3), such as forceps, to remove the stent (1000) from the lumen (1).

Although the present disclosure has been illustrated and described with reference to specific preferred embodiments, the present disclosure is not limited thereto, and various modifications and changes may be made by those skilled in the art without departing from the spirit and scope of the present disclosure.

### <Description of the Reference Numerals in the Drawings>

| | | | |
|---|---|---|---|
| 100: | Cylindrical body | 110: | Wire |
| 120: | Space portion | 130: | Bent portion |
| 200: | Pull string | 210: | First knotted portion |
| 220: | Second knotted portion | 230: | Gap |
| 1000: | Stent | | |

## Claims

1. A stent comprising a cylindrical body (100) formed such that shape memory alloy wires (110) are woven or crossed in a mesh form to have a hollow cylindrical shape so that multiple space portions (120) are formed between the wires (110) and multiple bent portions (130) are formed along a circumference of the cylindrical body (100) at each of opposite ends thereof, and a pull string (200) installed at one end of the cylindrical body (100), the stent being configured to be deployed at a lesion site (1a) where stenosis or occlusion has occurred in a lumen (1) in a body and to expand the lesion site (1a), **characterized in that** the pull string (200) consists of a single strand,
one end of the pull string (200) is tied to the wire (110) of one of the bent portions (130) to form a first knotted portion (210),
an opposite end of the pull string (200) is woven through the spaces (120) of the multiple bent portions (130) along the circumference of the cylindrical body (100) so as to be positioned adjacent to the first knotted portion (210), and then protrudes outward from the cylindrical body (100), and
the opposite end of the pull string (200) protruding outward from the cylindrical body (100) is tied to form a second knotted portion (220).

2. The stent of claim 1, **characterized in that** the second knotted portion (220) comprises multiple second knotted portions (220) formed at predetermined intervals along a longitudinal direction of the pull string (200) protruding outward from the cylindrical body (100).

3. The stent of claim 1, **characterized in that** a gap (230) is formed between the one end of the cylindrical body (100) and the second knotted portion (220) adjacent thereto.
